Europäisches Patentamt

European Patent Office

Office européen des brevets

(19)

(11) Publication number: **0 061 348**
A1

(12)

# EUROPEAN PATENT APPLICATION

(21) Application number: **82301516.9**

(22) Date of filing: **23.03.82**

(51) Int. Cl.³: **A 61 K 7/043**
**C 09 D 3/00, C 08 L 1/18**

(30) Priority: **23.03.81 US 246836**

(43) Date of publication of application:
**29.09.82 Bulletin 82/39**

(84) Designated Contracting States:
**AT BE CH DE FR GB IT LI LU NL SE**

(71) Applicant: **A.H. ROBINS COMPANY, INCORPORATED**
**1407 Cummings Drive**
**Richmond Virginia 23220(US)**

(72) Inventor: **Benkendorf, Sol**
**2403 Mountainbrook Drive**
**Richmond Virginia 23233(US)**

(72) Inventor: **Calamito, Frank Angelo**
**1 Babbling Brook Road**
**Sufferin New York 10901(US)**

(72) Inventor: **Zaccaria, Carmine Mark**
**574 Chestnut Street**
**Westwood New Jersey 07675(US)**

(74) Representative: **Kearney, Kevin David Nicholas et al,**
**KILBURN & STRODE 30 John Street**
**London, WC1N 2DD(GB)**

(54) Nitrocellulose lacquer composition containing gelatin and acrylic copolymers.

(57) An improved nitrocellulose lacquer containing small amounts of water-soluble gelatin preferably of molecular weight in the range from 15,000 to 250,000 and an acrylic copolymer and a novel process for dispersing and incorporating the gelatin are disclosed.

EP 0 061 348 A1

# NITROCELLULOSE LACQUER COMPOSITION
# CONTAINING GELATIN AND ACRYLIC COPOLYMERS

## Technical Field

This invention relates to novel lacquer compositions which afford desirable improvements in the durability and hardness of the resulting coatings. More particularly, this invention is concerned with nitrocellulose-resin based lacquers which contain small amounts of both a high molecular weight, water-soluble gelatin and an acrylic copolymer and conventional additives. The lacquers are particularly useful for human nail coatings. The invention also extends to a process for preparing the lacquers which involves an intermediate to which the invention also extends which intermediate is a solid dispersion of water-soluble gelatin in nitrocellulose.

## Prior Art

As is generally known, various base coatings have been prepared and marketed in the past for nail surfacing compositions, or nail varnish and various attempts have been made to improve the general performance of coatings which have nitrocellulose as the primary film-forming base.

Addition of finely particulate water-insoluble albuminoids such as keratin to nail varnish containing cellulose nitrate and natural or synthetic resins has been disclosed in US Patent 3,483,289.

U.S. Patent 3,907,580 discloses schleroprotein derivatives having such as a collagen hydrolyzate of animal origin with a pH in the range of about 3 to 7.5 in combination with nitrocellulose and an (aryl)sulphonamide-formaldehyde resin.

French Patent 1,572,598 utilizes an alcohol- soluble protein (collagen) which is specified to be a hydrolyzed gelatin with a molecular weight range of up to 10,000, preferably 500-1000.

The use of methacrylate resins in a cellulose nitrate based fingernail enamel composition is disclosed in US Patent 2,195,971.

While the incorporation of lacquer-insoluble pigments via plasticized nitrocellulose sheet is a known procedure, W.C.Doviak "Cosmetics, Science and Technology" 2nd Ed. Vol 2, p. 528(1972), Wiley Interscience Ed. M.S. Balsam et al., this method of dispersing water-soluble gelatin has not heretofore been disclosed.

Prior to the present invention, the combined use of a water-soluble gelatin having molecular weight in the range from 15,000-250,000 and an acrylic copolymer to drastically modify and improve the appearance and performance of coatings having the film formers utilized in this invention has not been disclosed. Moreover, in addition to the novelty of the combination, the method of preparation is also new.

First trials to make a lacquer containing gelatin of the nature envisioned: i.e. water-soluble gelatin of molecular weight in the range from 15,000-250,000 derived from mixture of bone and hide, in combination with the acrylic copolymer met with failure when methods of incorporating and dispersing collagenous materials known from the prior art were tried. To sum up these unsuccessful attempts, the water-soluble gelatin always settled out of the lacquer even after the most vigorous and efficient types of stirring were employed.

## Disclosure of the Invention

As a result of persistent efforts and consideration, methods were found to carry out this invention. These methods utilize high shear grinding of the water-soluble gelatin having a molecular weight in the range from 15,000-250,000 This dispersion of gelatin, which is colloidal in nature, can be obtained by conventional means for forming colloid suspensions such as by the use of ball mills or roller mills. The gelatin is predispersed in the solvents, resins, and plasticizers, which are used in the formulation. One method used to carry out the invention employs coplasticizing in intimate admixture the water-soluble gelatin having molecular weight in the range from 15,000-250,000 and the nitrocellulose. Preparation of the lacquer of this invention requires predispersion of the gelatin, believed to be of colloidal size, in a solid plasticized nitrocellulose mass, a procedure not previously known in the art.

The present invention provides improved nitrocellulose-based lacquer compositions especially suitable for application as surface coatings for human nails i.e. as nail varnish. The lacquer compositions are free-flowing liquids which may be applied to fingernails and toenails in conventional manner; for example, with a small brush, which provide improved coatings when they harden, being firmly adhering, durable coatings which remain intact for periods of time of at least 5-6 days under average exposure to the environment at home and/or in an office. The cured coatings have a "wet look" of freshly applied lacquer which is retained for extended periods.

4.

0061348

The lacquers of this invention are comprised on an overall basis of a primary film former which is nitrocellulose, two or more secondary film formers, one of which is always acrylic copolymer present in proportion of at least about 10% and no more than about 70% by weight of the total secondary film formers, water-soluble animal gelatin of molecular weight in the range from 15,000-250,000 suitable plasticizers and liquid carriers therefor including solvents, couplers and diluents, all of which liquid, carriers are for the most part evaporants. Suspension aids may also be added.

The process according to the present invention for preparing the nitrocellulose lacquer composition involves intimately predispersing the gelatin in a substantial portion of the nitrocellulose by plasticizing the mixture with a suitable plasticizer and lacquer solvents to form a thick plastic mass and thereafter roller-milling the plastic mass to form a gelatin-nitrocellulose sheet which is cooled and converted into particulate form,, the resultant particulate material being mixed with other lacquer components.

The improvements in properties of adherence to surfaces, durability and the "wet look" are due to the combined effect of the water-soluble gelatin having molecular weight in the range from 15,000 to 250,000 and the acrylic copolymer.

As will be readily realized, the process for preparing the lacquer also provides a novel intermediate, the solid dispersion of water-soluble gelatin in nitrocellulose. The process for preparing this intermediate comprises mixing lacquer components comprised of nitrocellulose, a plasticizer, a water-soluble gelatin having a molecular weight in the range from 15,000 to 250,000 and lacquer solvents to form a plastic mass and thereafter working and spreading the mass under compression and squeezing. action, preferably between rollers, to disperse the gelatin and drive off the solvents.

It is therefore an object of the present invention to provide novel lacquer compositions comprised of nitrocellulose primary film-former, two or more resinous secondary film-formers, one of which is always acrylic copolymer, a small amount of a water-soluble animal gelatin having molecular weight in the range from 15,000 to 250,000 suitable plasticizers and other conventional lacquer additives and carriers which are especially suitable for human nail coatings.

When the proportion of acrylic copolymer based on the total secondary film-formers is below about 10%, there is no significant improvement in durability of the dried coatings. When the proportion of acrylic copolymer based on the total secondary film-formers is above about 70%, the lacquer is too thick and is hard to control.

Another object of this invention is to provide human nail lacquer compositions which cure to coatings of exceptionable hardness and durability and which retain the high gloss appearance of the "wet look" of freshly-applied lacquer.

Another object is to provide a process for preparing uniform and stable nail lacquer compositions containing intimately dispersed water-soluble gelatin having molecular weight in the range from 15,000 to 250,000 always in combination in the lacquer with an acrylic copolymer.

The lacquers of the present invention suitable for human nail coverings and having improved hardness, durability and appearance preferably comprises as a primary film-former, nitrocellulose, two or more resinous secondary film-formers, one of which is always acrylic copolymer, water-soluble animal gelatin having a molecular weight in the range from 15,000 to 250,000, optionally pigments and colouring agents; plasticizer and a suspending agent, all in liquid carrier vehicle comprising couplers, diluents and volatile solvents. The acrylic copolymer and water-soluble gelatin serve as modifiers or activators.

The nail lacquer in a preferred form contains, based on overall weight %, from 10-25%, preferably 15-20% of nitrocellulose; from 1-3%, preferably 1.5-2.5% of acrylic copolymer resin; from 5 to 10%, preferably 7 to 8% of secondary film-forming resin other than acrylic copolymer or a synthetic modified resin; from 0.25-1.0% preferably 0.4 to 0.8% of water-soluble animal gelatin having molecular weight in the range from 15,000 - 250,000; 4-8%, preferably 5-7% of plasticizer, the balance being liuqid carrier vehicle including couplers, solvents and diluents.

The nitrocellulose used is preferably lacquer grade nitrocellulose, preferably of the type "RS" or "SS" and in particular nitrocellulose type RS 1/4 seconds, nitrocellulose type RS 1/2 seconds and nitrocellulose RS type 5/6 seconds. The "RS" type nitrocelluloses are preferred, type RS 5/6 second being preferred over all others.

In addition to acrylic copolymer which is always present, the secondary film forming resins suitable for the practice of this invention include one or more of the following synthetic resinous materials; arylsulphonamide formaldehyde, vinyl, vinylidene, alkyd, epoxy, melamine, phenolic, polyester, polyurethane, urea formaldehyde, polyethylene, polystyrene and polypropylene and the natural dammar resin. The synthetic resins are preferred and of these the preferred secondary film-forming resin used in conjunction with acrylic copolymer is an arylsulphonamide formaldehyde resin. Suitably, the aryl-sulphonamide formaldehyde resin is a naphthyl, phenyl or substituted phenyl sulphonamide. Preferably, the arylsulphonamide resin used in lacquers of this invention is toluenesulphonamide formaldehyde resin.

Plasticizing agents usable in the lacquers include tricresyl phosphate, benzyl benzoate, tributyl phosphate, butyl acetyl ricinoleate, glyceryl acetyl ricinoleate, dibutyl phthalate, dibutyl glycolate, dioctyl phthalate, butyl stearate, tributoxy ethyl phosphate, triphenyl phosphate, triethyl citrate, tributyl citrate, tributyl acetyl citrate, 2-hexyl triethyl acetyl citrate, dibutyl tartrate, dimethoxy ethyl phthalate, di-isobutyl phthalate, diamyl phthalate, camphor and various mixtures thereof.

Of these, dibutylphthalate plasticizer is preferred, preferably along with a small amount of camphor.

The gelatins suitable for practicing the invention are preferably the Type B gelatins which are prepared by alkaline hydrolysis of animal collagen, preferably by alkaline hydrolysis of bone or hide and combinations thereof under conditions which provide gelatin of molecular weight in the range from 15,000 to 250,000. The gelatin preferred in this invention is derived from bone and gelatin, preferably about 50-80% bone gelatin and 20-50% hide gelatin and especially preferred is gelatin prepared by 65-75% from bone and 25-35% from hide.

By acrylic copolymer is meant a polymerized combination selected from among acrylic, methacrylic, 2-hydroxy ethyl methacrylic acids, their esters, amides and alkali metal and ammonium salts and the like. Examples of suitable combinations are:

1) acrylamide-sodium acrylate copolymer
2) acrylate-acrylamide copolymer
3) acrylate-ammonium methacrylate copolymer
4) acrylates-copolymer
5) acrylic-acrylate copolymer which is an acrylic acid and methacrylic acid or an ester.

The one preferred is acrylates-copolymer, which is a polymer of two or more monomers consisting of acrylic acid and methacrylic acid and simple esters thereof.

Among the solvents usable in the compositions of the lacquers, a few in particular are cited: ethyl acetate, butyl acetate, amyl acetate, methylacetate, methylisobutylketone, methylethyl ketone, methyl glycol acetate and mixtures of such solvents. Preferably, a mixture of butyl and ethyl acetates is used in the mixture.

9.                                    0061348

Among the so-called couplers, the lower molecular weight alkanols may be used such as ethanol, propanol, isopropyl alcohol, n-butyl alcohol and mixtures thereof. Preferably, a mixture of isoproyl alcohol and n-butyl alcohol are used.

As diluents, aromatic or aliphatic hydrocarbons such as toluene or heptane may be used or a mixture thereof. Preferably, the diluent used is toluene. Inorganic pigments such as titanium dioxide, coloured polymeric material and dyes may be used for colour effects.

In general, the process for preparing the lacquers of this invention comprises working a mixture comprising water-soluble gelatin, nitrocellulose plasticizer and lacquer solvents under compression to intimately disperse the gelatin, driving off the solvents to form a solid predispersion comprising gelatin dispersed in nitrocellulose and plasticizer and thereafter combining the solid predispersion in particulate form with lacquer components among which are two or more secondary film formers, one of which is always acrylic copolymer.

More specifically the process for the preparation of a nitrocellulose lacquer containing water-soluble gelatin and acrylic copolymer comprises the steps of

1) mixing lacquer components comprising water-soluble gelatin of molecular weight in the range from 15,000 to 250,000, a portion of the total plasticizer and sufficient lacquer solvents to give a high viscosity liquid,

10.                                    **0061348**

2) working the high viscosity liquid obtained in
   step 1 under compression and squeezing motion
   preferably on a roll-mill, to disperse the
   gelatin and form a solid predispersion
   comprising nitrocellulose, gelatin and
   plasticizer, preferably as a sheet or ribbon,

3) converting to particulate form the solid from
   step 2), preferably by grinding in at least
   a portion of lacquer liquids and

4) mixing and/or further converting to particulate form solids from step 3) with the balance
   of the lacquer ingredients which include
   acrylic copolymer and another secondary film-
   former.

A general description of the individual steps of
the process follows.

In step 1) the gelatin is preferably mixed with
about 4 to 30 times, preferably about 7-8 times, its own
weight of nitrocellulose and about 1 to 6 times, preferably about 1 to 2 times, its own weight of plasticizer,
preferably dibutylphthalate, together with sufficient
lacquer solvents and liquids to form a high viscosity
liquid. The lacquer solvents preferably are used in the
same or about the same ratio as in the basic overall
finished formulation used to make a particular lacquer.
Obviously, because the solvents are evaporated for the
most part in the next step, many equivalent variations
will be apparent, including the use of some which are
not used in quantity in the final product.

11.

0061348

In step 2) the resultant high viscosity liquid is preferably worked by spreading the mass from step 1) under compression and squeezing action, preferably between water-cooled rollers of a roller mill in repetition until the gelatin has become intimately dispersed. Illustratively, about 3 or 4 passes through a 2-roll water-cooled roller mill will be sufficient.

In step 3) the gelatin-nitrocellulose dispersion is preferably converted to particulate form in at least a portion of the lacquer liquids. The proportion of lacquer liquid to the total lacquer liquids may vary over a wide range up to and including the total liquids. The nitrocellulose-gelatin dispersion comprises three major components ranging in proportions, in parts by weight, of 3-17 of gelatin, preferably about 10 of gelatin; about 69-81 of nitrocellulose, preferably about 75 of nitrocellulose; and about 14-16 of plasticizer, preferably about 15 of plasticizer. These ranges are exclusive of small amounts of solvents which remain trapped in the solid sheet.

In step 4) a clear lacquer base comprising 75% of the solvents and diluents portion of the formula are preferably placed in a mixing tank. Under agitation with a high-shear mixer, nitrocellulose is first added and dissolved followed by addition of the gelatin dispersion prepared in step 3), the secondary film-forming resins including acrylic copolymer and remaining plasticizer and suspending agent. Sufficient mixing time is allowed to ensure homogenuity. Colour dispersions are optionally added, viscosity is adjusted to that required and the lacquer is filtered.

Obviously, many variations in order of addition will become apparent to those skilled in the art.

BEST MODE OF CARRYING OUT THE INVENTION

The invention may be put into practice in various ways and a number of specific embodiments will be described to illustrate the invention with reference to the following examples in which Examples 1 and 5 are embodiments of the invention and 2 to 4 are comparative.

## Example 1
### (Gelatin and Acrylic Copolymer)

A lacquer composition having the following overall composition was prepared by the method following.

| Ingredient | Weight % of Ingredient In Finished Lacquer |
|---|---|
| Nitrocellulose, RS 5/6 Sec | 17.50 |
| Toluenesulfonamide formaldehyde resin | 7.50 |
| Gelatin (a) | 0.60 |
| Dibutyl phthalate | 5.00 |
| Acrylates-copolymer | 2.00 |
| Camphor | 0.55 |
| Butyl acetate | 17.50 |
| Ethyl acetate | 7.50 |
| Isopropyl alcohol | 5.00 |
| n-Butyl alcohol | 0.55 |
| UV Absorber | 0.05 |
| Toluene | 36.25 |
| | 100.00 |

(a) Manufacturer's specification for derivation of gelatin is 70% bone, 30% hide. The molecular weight is 15,000-250,000.

### (Predispersion of Gelatin in Plasticized Nitrocellulose)

13.

0061348

A solid dispersion of gelatin in nitrocellulose was prepared as follows: a charge of all the gelatin, 9 times its own weight of nitrocellulose, 3 times its own weight of dibutylphthalate, together with sufficient lacquer solvents preferably in the overall proportions contained in the finished lacquer to wet the mass was mixed in a High Shear Cowles mixer to give a high viscosity liquid. The mass was charged onto the rolls of a highspeed, water-cooled roller mill and the high viscosity liquid was recycled through the roll mill until the maximum dispersion was obtained.

### (Preparation of Lacquer Base)

To 70% of the solvents in the ratio required in the above formulation held in a tank under high-shear agitation (i.e. Hochmeyer (Registered Trade Mark) dispenser) was added the remainder of nitrocellulose not used in making the gelatin nitrocellulose predispersion or chip. When the nitrocellulose had dissolved, the toluene sulphonamide formaldehyde resin, the remainder of the dibutyl phthalate, camphor and UV absorber were added in sequence. Agitation was continued until the ingredients were substantially dissolved. The solution was filtered to remove traces of impurity and the acrylates-copolymer was added under agitation.

### (Preparation of Finished Lacquer)

The gelatin-nitrocellulose dispersion prepared above was dispersed in the remaining 30% of the solvents under high shear agitation and added to the foregoing lacquer base under agitation. The lacquer was filtered.

Comparative Examples 2-4

Lacquer compositions for comparative purposes were prepared following:

| Ingredient | Example 2 Gelatin- (No Acrylate) | Example 3 Acrylate- (No Gelatin) | Example 4 (No Gelatin- No Acrylate) |
|---|---|---|---|
| Nitrocellulose, RS 1/2 Sec. | 17.50 | 17.50 | 17.50 |
| Toluenesulfonamide formaldehyde resin | 7.50 | 7.50 | 7.50 |
| Gelatin (a) | 0.60 | - | - |
| Dibutylphthalate | 5.00 | 5.00 | 5.00 |
| Acrylates-copolymer | - | 2.00 | - |
| Camphor | 0.55 | 0.55 | 0.55 |
| Butyl acetate | 17.50 | 17.50 | 17.50 |
| Ethyl acetate | 7.50 | 7.50 | 7.50 |
| Isopropyl alcohol | 5.00 | 5.00 | 5.00 |
| n-Butyl alcohol | 0.55 | 0.55 | 0.55 |
| UV Absorber | 0.05 | 0.05 | 0.05 |
| Toluene | 38.25 | 36.85 | 38.85 |
| Total | 100.00 | 100.00 | 100.00 |

(a) Same as Example 1.

The lacquer of Example 2 was prepared by the same procedure as Example 1, except no acrylic copolymer was added. The composition of Example 3 required no gelatin dispersion and was prepared as described in Example 1 under "Preparation of Lacquer Base," using all the solvent, plasticizer and nitrocellulose in that step and the lacquer base was the finished lacquer. The composition of Example 4 having neither gelatin nor acrylic monomer was prepared as in Example 3, except no acrylic was added.

Example 5

A lacquer composition having the following overall composition was prepared by the method following:

| Ingredient | Weight % of Ingredient In Finished Lacquer |
|---|---|
| Nitrocellulose, RS 1/2 Sec. | 17.50 |
| Toluene sulphonamide formaldehyde resin | 7.00 |
| Gelatin (a) | 0.66 |
| Dibutyl phthalate | 5.25 |
| Acrylates-copolymer | 2.00 |
| Camphor | 1.00 |
| Butyl acetate | 20.00 |
| Ethyl acetate | 7.00 |
| Amyl acetate | 5.00 |
| Isopropyl alcohol | 5.00 |
| n-Butyl alcohol | 1.00 |
| UV Absorber | 0.05 |
| Toluene | 25.50 |
| Stearalkonium hectorite (Clay suspension agent) | 1.04 |
| Pigment dispersion | 2.00 |
| Total | 100.00 |

(a) Same as Example 1.

Predispersed gelatin-nitrocellulose chip was prepared as in Example 1. Lacquer base was also prepared as in Example 1 except a small portion of the nitrocellulose and solvent was withheld and used to mix with and form a suspension with the clay suspension agent. The acrylic copolymer was then added to this suspension which was in turn added to the lacquer base. The lacquer was then finished as in Example 1 by adding the gelatin-nitrocellulose dispersion into the lacquer base and finally adding the pigment dispersion and filtering.

### Test Procedure

General Description: Preformed acrylic "Pinback" paintable nails were twice coated with nail lacquer using a small brush, dried for 24 hours and tumbled with sand in tubes mounted on a rotating bar for a given period of time. The coatings are then observed, scored and evaluated to obtain a "Wear Index." The lower the "Wear Index," the better the coating.

Coating Procedure: Blank#3 "Pinback" acrylic nails used in the trade to display various nail polishes were fixed in place in rows via the raised knob of the back of the nails so that the rounded surface was face up on a 1/8" (3.2 mms) thick flat arcylic sheet. The raised knobs fitted into holes drilled in an acrylic sheet. The nails were then painted using a nail polish brush, dipping from a small nail polish bottle wnich had been shaken first, always dipping the brush once per nail and uniformly coating the surface and edge. The nails were painted a second time allowing a suitable amount of time between coats for drying and a minimum of 24 hr after the last coat. The coated nails were removed from the acrylic sheet for testing.

## Abrasion Test Apparatus and Procedure

Capped 50 ml tubes (1" (2.5 cms) I.D.) were loaded with 5 g sea sand (Fisher Scientifiq, washed and ignited) and 5 coated nails. The tubes were mounted vertically with clamps to a mechanically rotatable bar. The bar with tubes attached containing the sand and nails was rotated for 18 hours at 20 rpm, each revolution moving sand and nails from one end of the tube to the other. A total of 15 painted nails were used in each evaluation.

## Test Evaluation Procedure

The nails were removed from the tubes and separated from the sand. Each nail was visually observed individually and given a grade from 0 to 5 as follows:

O - unabraded (control)

1 - slight body and edge wear

2 - slight to moderate body and edge wear

3 - moderate body wear and loss of shine with slight chipping

4 - moderate to severe body wear, loss of shine, chipping without bare spots*

5 - severe body wear with bare spots* and extensive edge wear

*bare spot - an area where the coating has been worn away, revealing the underlying surface of the original unpainted nail.

The "Wear Index" was determined by multiplying the number of nails at each grade level by the grade number, adding the total and dividing by the total number of nails treated. Results of tests on 15 nails for each of Examples 1-4 are in Table 1.

## Table 1
### Abrasion Resistance Tests

| Example No. | Significant Characteristic of Lacquer Composition | No. out of 15 at Each Grade Level | | | | | | Wear Index |
|---|---|---|---|---|---|---|---|---|
| | | 0 | 1 | 2 | 3 | 4 | 5 | |
| 1 | Gelatin & acrylic | 0 | 6 | 5 | 4 | 0 | 0 | 1.86 |
| 2 | Gelatin - no acrylic | 0 | 3 | 5 | 5 | 2 | 0 | 2.40 |
| 3 | Acrylic - no gelatin | 0 | 0 | 3 | 8 | 4 | 0 | 3.07 |
| 4 | No gelatin - no acrylic | 0 | 0 | 5 | 10 | 0 | 0 | 2.67 |

18.                                    0061348

As can be observed, the "Wear Index" obtained with the
lacquer having the combination of gelatin and acrylic copolymer was superior to lacquers having gelatin alone,
acrylic copolymer alone or lacquer having neither gelatin
nor acrylic copolymer.

**0061348**

CLAIMS

1. A lacquer composition comprising a nitrocellulose primary film-former, and a secondary film-former and suitable plasticizers and liquid carriers therefor, characterised in that the secondary film-former comprises two or more resinous secondary film-formers, one of which is always acrylic copolymer, the said acrylic copolymer being present in proportion of at least about 10% and no more than about 70% by weight based on the total secondary film formers and in that the composition includes a water-soluble animal gelatin having a molecular weight in the range from 15,000 to 250,000

2. A lacquer composition as claimed in claim 1 in which the gelatin is 50-80% bone gelatin and 20-50% hide gelatin.

3. A lacquer composition as claimed in claim 1 or claim 2 in which the said gelatin content is 0.25 to 1.0 wt % and the acrylic copolymer content is 1 to 3 wt %.

4. A lacquer composition as claimed in claim 1 or claim 2 in which the gelatin content is 0.4 to 0.8 wt % and the acrylic copolymer content is 1.5 to 2.5 wt %.

5. A laquer composition as claimed in anyone of the preceding claims in which the resinous secondary film former used in addition to the acrylic copolymer is toluene sulphonamide formaldehyde resin.

6. A process for preparing a nitrocellulose lacquer containing gelatin and acrylic resin which comprises working a mixture comprising gelatin, nitrocellulose, plasticizer and lacquer solvents under compression to intimately disperse the gelatin, driving off the solvents to form a predispersion comprising gelatin dispersed in nitrocellulose and plasticizer and thereafter combining the said predispersion in particulate form with lacquer components among which are two or more resinous secondary film formers, one of which is always acrylic copolymer.

7. A process for the preparation of a nitrocellulose lacquer containing water-soluble gelatin which comprises the steps of

1) mixing lacquer components comprising soluble gelatin of molecular weight in the range from 15,000 to 250,000, and a portion of the total plasticizer lacquer solvents to give a high viscosity liquid,

2) working the high viscosity liquid obtained in step 1 under compression and squeezing motion to disperse the gelatin and form a predispersion comprising nitrocellulose, gelatin and plasticizer,

3) converting the mass from step 2 to particulate form and

4) mixing or converting further to particulate form or both mixing and converting further to particulate form the mass from step 3 with the balance of the lacquer ingredients among which are two or more resinous secondary film formers, one of which is always acrylic copolymer.

8. A process as claimed in claim 7 in which in step 2 the high viscosity liquid is worked on a roll mill.

9. A process as claimed in claim 7 or claim 8 in which in step 3 the mass is converted to particulate form by grinding in at least a portion of the lacquer liquids.

10. A process as claimed in anyone of claims 7 to 9 in which the amount of the said gelatin added based on the weight of finished lacquer is 0.25 to 1.0% and the amount of acrylic copolymer is 2 to 6%.

11. A process as claimed in anyone of claims 7 to 9 in which the amount of the said gelatin added based on the weight of the finished lacquer is 0.4 to 0.8% and the amount of acrylic copolymer is 3 to 5%.

12. A colloidal dispersion of water-soluble animal gelatin in plasticized nitrocellulose, the said animal gelatin being present in an amount of 3 to 17 wt %, and having a molecular weight in the range from 15,000 to 250,000.

13. A process for preparing a dispersion of water-soluble gelatin in nitrocellulose which comprises mixing lacquer components comprising nitrocellulose, a plasticizer, a water-soluble gelatin having molecular weight in the range from 15,000 - 250,000 and lacquer solvents to form a plastic mass and thereafter working and spreading the mass under compression and squeezing action to disperse the gelatin and drive off the solvents.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y,D | FR-A-1 572 598 (FARBARGE)<br>* Whole document * & GB - A - 1 182 875<br><br>--- | 1-13 | A 61 K 7/043<br>C 09 D 3/00<br>C 08 L 1/18 |
| Y,D | US-A-3 483 289 (MICHAELSON et al.)<br>* Whole document *<br><br>--- | 1-13 | |
| Y,D | US-A-3 907 580 (VAN HAM)<br>* Whole document *<br><br>--- | 1-13 | |
| Y | CHEMICAL ABSTRACTS, vol. 93, no. 18, November 1980, pages 345,346, no. 173608c, Columbus, Ohio, USA<br>& RO - A - 65293 (INTREPRINDEREA DE PRODUSE COSMETICE MIRAJ) 03-08-1978 * Abstract *<br><br>--- | 1-13 | |
| Y | FR-A-1 267 983 (NORTHAM WARREN CORP.)<br>* Page 6, column 2, example p *<br><br>--- | 1-13 | TECHNICAL FIELDS SEARCHED (Int. Cl. 3)<br><br>A 61 K 7/00<br>A 45 D 31/00<br>C 08 L 1/00<br>C 09 D 3/00 |
| A | FR-A- 319 926 (SOC. INDUSTRIELLE DE CELLULOSE)<br>* Whole document *<br><br>--- | 12,13 | |

The present search report has been drawn up for all claims

| Place of search | Date of completion of the search | Examiner |
|---|---|---|
| THE HAGUE | 28-06-1982 | BENZ K.F. |

**European Patent Office**

## EUROPEAN SEARCH REPORT

Application number

EP 82 30 1516

| | DOCUMENTS CONSIDERED TO BE RELEVANT | | Page 2 |
|---|---|---|---|

| Category | Citation of document with indication, where appropriate, of relevant passages | Relevant to claim | CLASSIFICATION OF THE APPLICATION (Int. Cl. 3) |
|---|---|---|---|
| A | CHEMICAL ABSTRACTS, vol. 86, no. 18, 2nd May 1977, page 107, no. 123247u, Columbus, Ohio, USA & JP - A - 77 04570 (MITSUBISHI PAPER MILLS, LTD.) 13-01-1977 * Abstract * | 12,13 | |
| | ----- | | |

TECHNICAL FIELDS
SEARCHED (Int. Cl. 3)

The present search report has been drawn up for all claims

| Place of search | Date of completion of the search | Examiner |
|---|---|---|
| THE HAGUE | 28-06-1982 | BENZ K.F. |